# EUROPÄISCHE PATENTSCHRIFT

(11) **EP 1 037 046 B1**
(45) Veröffentlichungstag und Bekanntmachung des Hinweises auf die Patenterteilung: **19.12.2001**
(21) Anmeldenummer: 00103198.8
(22) Anmeldetag: 17.02.2000
(51) Int. Cl.: G01N 33/20, G01N 33/00

(54) **Verfahren zum Bestimmen des Quecksilbergehaltes in kontaminierten Stahlrohren und/oder -armaturen**
Apparatus for determining the mercury content in contaminated steel pipes and/or armatures
Appareil pour déterminer le contenu en mercure dans des tuyaux et/ou armatures en acier

(30) Priorität: 13.03.1999 DE 19911206
(43) Veröffentlichungstag der Anmeldung: 20.09.2000
(73) Patentinhaber: NET- Norddeutsche Energie Technik GmbH, 19258 Boizenburg (DE); BEB ERDGAS UND ERDÖL GMBH, D-30659 Hannover (DE)
(72) Erfinder: Ingenillem, Franz-Ulrich, 21220 Seevetal (DE); Sander, Wolfgang, 30916 Isernhagen (DE)
(74) Vertreter: Fleck, Thomas, Dr. Dipl.-Chem.

(56) Entgegenhaltungen:
- DE-A- 4 214 885
- DE-C- 19 506 875
- FR-A- 2 359 412
- US-A- 5 597 535
- US-A- 5 679 957

## Beschreibung

Die Erfindung betrifft ein Verfahren zum Bestimmen des Quecksilbergehaltes in kontaminierten Stahlrohrabschnitten und/oder -armaturen, bei denen es sich um Erdgasleitungen handelt.

Im Stand der Technik sind zwei Verfahren bekannt, nämlich das sogenannte "Glörfeld"- und das "Königswasser"-Verfahren. Letztere erfordern den Ausbau eines Teilstückes des Stahlrohres und den Transport der Probe in ein entsprechendes Chemielabor, die relativ komplizierte und aufwendige quantitative Quecksilberbestimmungen in der Regel mindestens 24 h erfordern.

Nach dem "Glörfeld"-Verfahren wird bei einer Temperatur zwischen 500 und 600° C das Quecksilber verdampft, Quecksilberverbindungen zum metallischen Quecksilber reduziert und die Quecksilberdämpfe mit Hilfe eines Trägergasstroms in ein Absorptionssystem geleitet. Nach Kondensation bzw.. Absorption an Goldfolie wird das Quecksilber mittels ICP-OES gemessen.

Bei der Durchführung des "Königswasser"-Verfahrens wird zunächst die Masse des zu untersuchenden Stahlrohres bestimmt und in ein Gewinderohr eingespannt, daß nur dia zu untersuchende Fläche von den Säuren berührt werden kann. Die Oberfläche wird dann mit einem Gemisch (3 Teile konzentrierte Salzsäure, 1 Teil konzentrierte Salpetersäure) geätzt. Nach einer Einwirkzeit von 20 Minuten wird das Säuremisch in einen Meßkolben überführt. Nach dem Trocknen wird die Masse des Prüfkörpers erneut bestimmt. Bei einem Prüfkörperdurchmesser von 20 mm muß der Masseverlust mindestens 0,2 g betragen (≙ 0,1 mm). Besteht der Verdacht, daß Quecksilber tiefer eingedrungen ist, muß die Behandlung wiederholt werden, wenn nötig bis zur vollständigen Auflösung des Prüfkörpers.

Der Quecksilbergehalt wird nach Reduktion durch flammenlose Atomabsorption bestimmt.

Darüber hinaus ist es aus Römpp Chemie Lexikon, 9. Aufl., 1992, S. 3737 bekannt, zur Spurenanalyse von Quecksilber die Atomabsoprtionsspektroskopie einzusetzen.

Ferner ist aus der DE-C1-195 06 875 ein geschlossenes Verfahren zum Bestimmen des Quecksilbergehaltes in Erdproben bekannt, bei dem eine konstante Luftströmung eingestellt und die Erdprobe solange erhitzt wird, um ein totales Verdampfen des Quecksilbers zu erzielen, wonach der Luftstrom mit dem davon aufgenommenen Quecksilber kontinuierlich abgesaugt und einer Meßeinrichtung zugeführt wird, um das gesamte freigesetzte elementare Quecksilber mittels Atomabsorptionsspektroskopie in der Gasphase zu messen und über einen kurzen Zeitraum aufzuzeichnen und integral der mg/Hg-Gehalt pro kg der Erdprobe zu errechnen.

Die Lehre der vorbekannten DE-A1-42 14 885 bezieht sich auf ein Quecksilber-Dekontaminationsverfahren, um von den Innenseiten demontierter Rohre und eventuell Armaturen, insbesondere von Gastransportleitung, Quecksilber zu entfernen. Es wird also das gesamte Quecksilber entfernt. Außerdem wird an den demontierten Rohren mit einer Beheizungseinrichtung gearbeitet, die das fest eingespannte Rohr ringförmig umgibt und darauf entlang bewegt wird. Insgesamt gesehen handelt es sich also um eine Reinigungsanlage, die an einem stationären Platz aufgebaut und betrieben wird.

Beide vorbekannten Lehren können also nicht im Feld vor Ort mobil eingesetzt werden.

Die bekannten Verfahren sind also nicht nur relativ zeitaufwendig, sondern zudem auch noch in wirtschaftlicher Hinsicht teuer.

Der Erfindung liegt deshalb die Aufgabe zugrunde, hier Abhilfe zu schaffen, d.h. das eingangs genannte Verfahren so zu verbessern, daß die Bestimmung des Quecksilbergehaltes in den Stahlrohren und -armaturen auf einfache und schnelle Weise wirtschaftlich durchgeführt werden kann. Ziel sollte es also sein, ein Verfahren für den Einsatz im Feld zur Verfügung zu stellen, das eine zeitnahe Bestimmung des Quecksilbergehaltes ermöglicht, wobei u.a. der Nachweis für die Erreichung bzw. Unterschreitung des von den Bergämtern geforderten Grenzwertes für Quecksilber in Stahl (5 ppm Hg) erbracht werden sollte.

Ausgehend von der Annahme, daß das Quecksilber im Mittel in etwa gleicher Verteilung auf bzw. in den ersten µm der Oberfläche des Rohres befindet, gilt, daß bei konstanten Temperatur-, Druck- und Strömungsbedingungen die Verdampfung des Quecksilbers im wesentlichen von der Oberfläche und damit von der Quecksilberkonzentration im Rohrabschnitt abhängig ist, so daß die Aufgabe erfindungsgemäß dann durch die im Anspruch 1 angegebenen Verfahrensstufen gelöst werden kann.

Zunächst wird erfindungsgemäß das Rohr mit Frischluft solange durchspült, bis sichergestellt, daß ein kompletter LuftwechseL stattgefunden hat. Dabei kann man sofort oder erst jetzt den Luftvolumenstrom auf einen vorgegebenen, konstanten Wert einstellen, beispielsweise von 30 m³/h, wobei sich bei einem Rohrinnendurchmesser von 205 mm eine Strömungsgeschwindigkeit von 0,25 m/sec ergibt. Bei dieser Strömungsgeschwindigkeit nimmt der Vorgang bei einem 50 m langen Rohrstück etwas über 3 min in Anspruch. Es dürfte einleuchten, daß der Fachmann bei anderen Größenverhältnissen entsprechende Anpassungen vornehmen kann. Die Strömungsgeschwindigkeit kann in einem geeigneten Rahmen verändert werden, wobei es sich als vorteilhaft erwiesen hat, daß diese unter 0,5 m/sec liegt und eben konstant gehalten wird.

Nach Einstellung einer konstanten, definierten Luftströmung an der Stahloberfläche des Rohres und Erfassung der Oberflächentemperatur kann durch Erhitzen einer definierten Rohroberfläche mit einem definierten Energieeintrag das dabei ausdampfende Quecksilber gemessen werden. Zwischen dem ausgedampften Quecksilber - sowohl als Höchstkonzentration (Spitzenwert/Peak) in mg Hg je m³ Luft, als auch als integraler Gesamtwert in mg ausgedampften Quecksilbers - und der Hg-Belastung der kontaminierten Rohre und/oder -armaturen besteht eine lineare Beziehung. Nach dem erfindungsgemäßen Erhitzen wird hierzu der Luftstrom abgesaugt und einer geeigneten Meßeinrichtung zugeführt. Elementares Quecksilber wird hier mittels Atomabsorptionsspektroskopie in der Gasphase ermittelt. Das Meßgerät wird hierzu anhand von Prüfgasen für den jeweiligen Meßbereich kalibriert- Der meßbare Wert liegt im µg-Bereich und deckt den für das hier beschriebene Feldmaßverfahren erforderlichen Meßbereich ab. Das erfindungsgemäße Verfahren dauert insgesamt maximal 5 bis 10 Minuten.

Weitere Vorteile und Merkmale gehen aus den Unteransprüchen hervor, die auch gemeinsam mit dem Hauptanspruch von erfindungsgemäßer Bedeutung sein können.

Im folgenden werden bevorzugte Ausführungsbeispiele zum besseren Verständnis der Erfindung aufgezeigt, die jedoch den Schutzumfang in keiner Weise einschränken sollen, da hier ausschließlich bevorzugte vorteilhafte Werte angeführt werden, die vom Fachmann im geeigneten Rahmen verändert werden können, ohne den Rahmen der Erfindung zu verlassen.

Beispiel für die Ermittlung des Hg-Gehaltes in vier verschiedenen Stahlrohren mittels des erfindungsgemäßen Feldmeßverfahren.
Rohr: 8 5/8", Innendurchmesser 205 mm, Wandstärke 6,9 mm, spezifisches Gewicht 37,1 kg/m, Länge 4.000 mm;
Frischluftdurchströmung des Rohres: 15 m³/h
Strömungsgeschwindigkeit: 0,126 m/sec
Dauer der Spülung: 1 min (min 32 sec)
Abstand des Brennerkopfes vom Rohranfang (in Strömungsrichtung): 3.500 mm
Positionierung der Brennerdüse: senkrecht auf das Stahlrohr
Abstand Brennerdüse von Stahlrohr: 6,0 mm
Brennertyp: Löt- und Wärmeeinsatz Z-F Gr. 6 Messer-Griesheim
Abstand der Meßentnahmestelle (Rohrmitte) vom Brennerkopf: 450 mm
Energiezufuhr: Acetylen, Vordruck 0,03 bar, Sauerstoff 2,7 bar,
Flammeneinwirkzeit: 30 sec; 0,3 kWh Energieeintrag
Flächen rotglühlend: außen 320 bis 490 mm² (ø 20 - 25 mm), innen 110 bis 180 mm² (ø 12 - 15 mm)
Oberflächentemperatur innen rotglühend bei Ende der Flammeneinwirkzeit: > 800° C;
Fläche innen größer 100° C bei Ende Flammeneinwirkzeit: ca. 150 cm²

| Meßwerte | Stand der Technik Labor | erfindungsgemäß im Feld | |
|---|---|---|---|
| Rohr | HG-Gehalt mgHg/kgSt | max. HG-Konz. mg/m³ Luft | Fig. |
| Z3 | 1,73 | 0,21 | 1 |
| TSw | 5,4 | 1,56 | 2 |
| F7 | 4,9 | 1,36 | 3 |
| N2 | 3,23 | 0,79 | 4 |

Als Meßgerät kann beispielsweis das "Mercury Vapour Monitor 791" der Fa. Products Manufacturing, Erm, Niederlande eingesetzt werden, das einen Meßbereich von 0 - 1999 µg/m³ abdeckt.

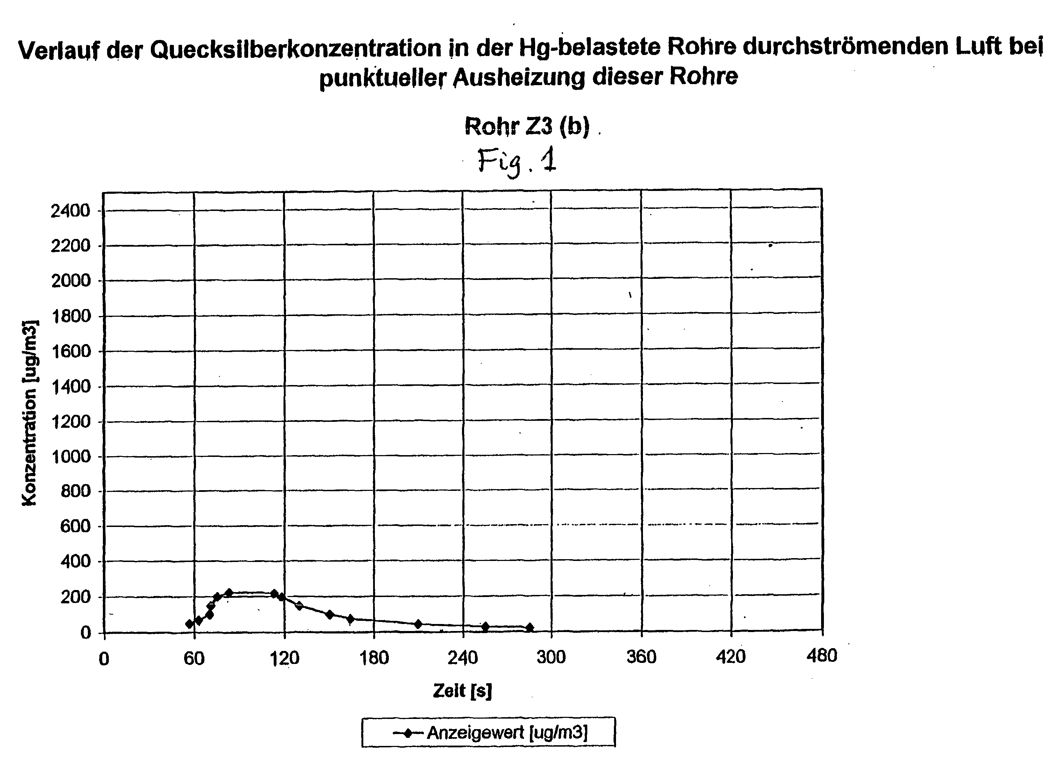

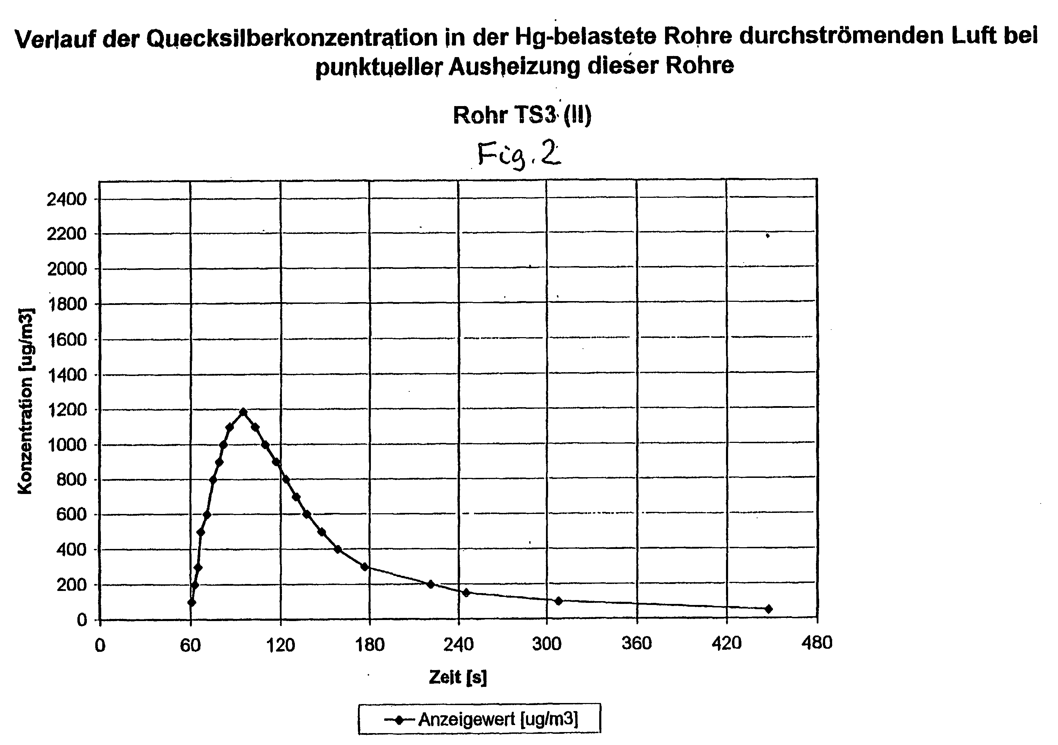

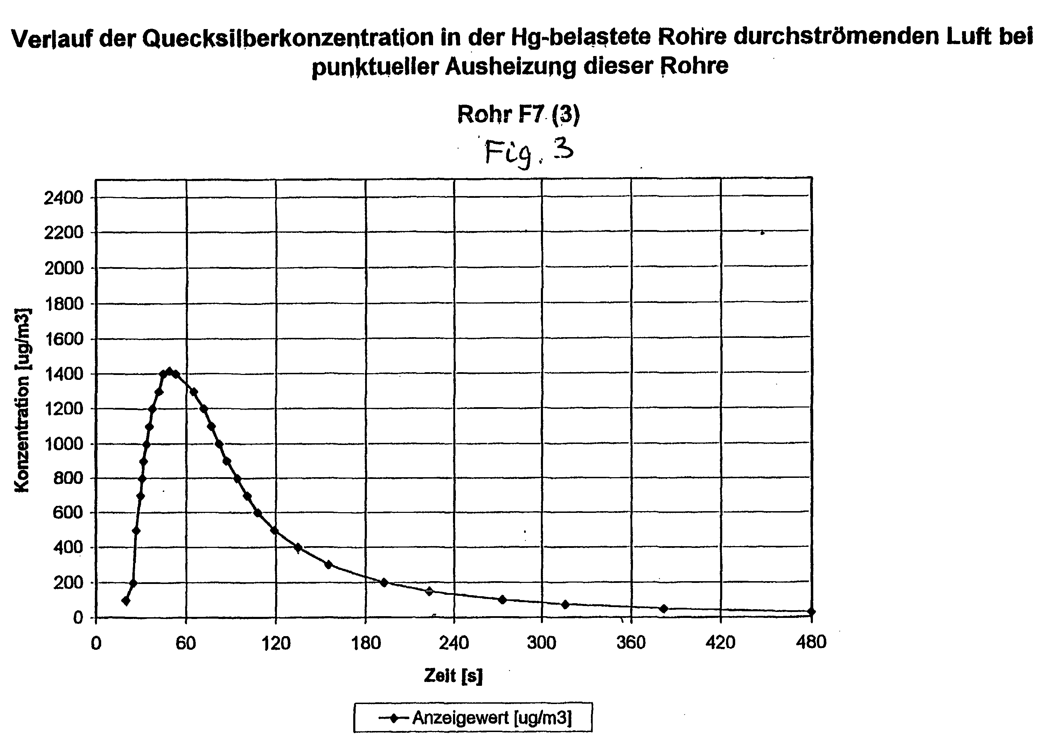

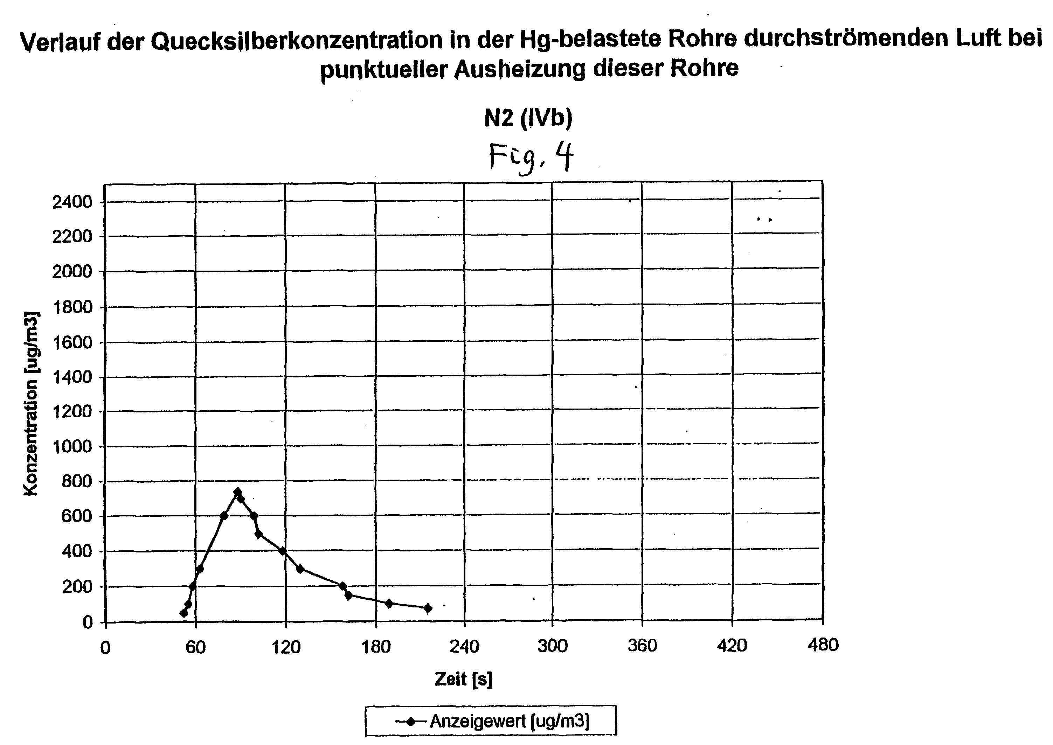

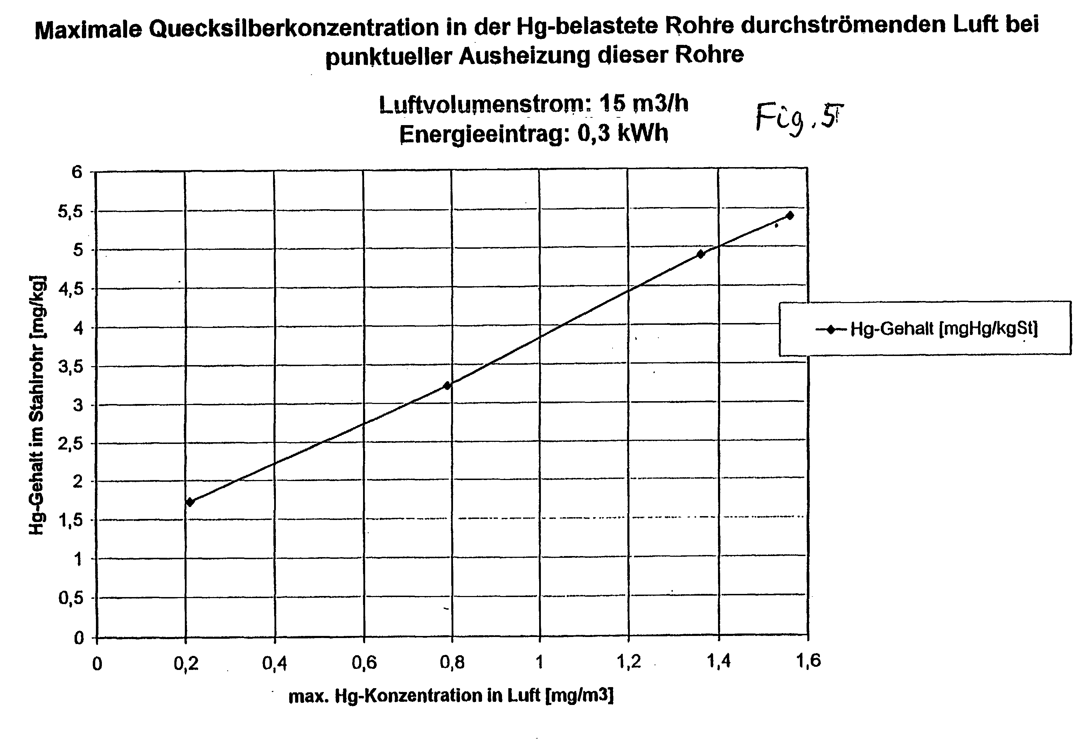

Wie deutlich aus Fig. 5 zu ersehen ist, besteht eine Linearität zwischen den im Labor im Stahlrohr und erfindungsgemäß im Eeld in der Luft ermittelten Hg-Werten, so daß man anhand letzterer den tatsächlichen Hg-Gehalt im Stahlrohr im Meßbereich extrapolieren kann.

Bei der Gegenüberstellung der Restgehalte an Quecksilber in den angeführten Testrohren mit den bei der punktuellen Erhitzung des Stahlrohrs ausgetriebenen Hg-Mengen ist im untersuchten Bereich eine lineare Zunahme der ausheizbaren Quecksilbermengen mit steigenden Hg-Gehalten im Stahl ersichtlich. Anhand des dargestellten Diagramms "Maximale Quecksilberkonzentration in der Hg-belastete Rohre durchströmenden Luft bei punktueller Ausheizung" gem. Fig. 5 ist somit bei der erfindungsgemäßen Methode die Einstufung von gereinigten Rohren bezogen auf den Zielwert der Reinigung von < 5 mgHg/kgST möglich.

## Patentansprüche

1. Verfahren zum Bestimmen des Quecksilbergehaltes in kontaminierten Stahlrohrabschnitten und/oder -armaturen, bei denen es sich um Erdgasleitungen handelt, die sowohl im Boden befindlich, als auch ausgebaut sein können, welches im Feld vor Ort durchgeführt wird mit folgenden Stufen:
a) anfängliches Spülen des kontaminierten, zu untersuchenden Stahlrohrabschnittes bzw. der -armaturen mit Frischluft, zur Erzielung eines vollständigen Luftwechsels in demselben bzw. denselben,
b) Einstellen einer vorgegebenen, konstanten laminaren Luftströmung,
c) Erhitzen einer definierten, punktuellen Außenfläche des Stahlrohrabschnittes bis zur Rotglut und seiner entsprechenden Innenfläche auf > 100°C bei konstantem Energieeintrag in den Rohrabschnitt, um ein Verdampfen des auf der Innenfläche und im Gefüge des Stahlrohrabschnittes angesammelten Quecksilbers zu erzielen,
d) kontinuierliches Absaugen des Luftstroms mit dem davon aufgenommenen Quecksilber und Zuführen zu einer Meßeinrichtung,
e) Messen des freigesetzten elementaren Quecksilbergehaltes im abgesaugten Luftstrom in an sich bekannter Weise mittels Atomabsorptionsspektroskopie in der Gasphase,
f) Aufzeichnen des freigesetzten Quecksilbergehaltes über einen kurzen Zeitraum und Feststellen des maximales Wertes,
g) Errechnen des mg/Hg-Gehaltes pro kg Stahlrohrabschnitt aufgrund der bestehenden Linearität zum mg/Hg-Gehalt in der festgelegten Luftstrommenge.

2. Verfahren nach Anspruch 1, **dadurch gekennzeichnet, daß** die konstante Luftströmung mittels eines Saugzuggebläses durchgeführt wird, welche insbesondere unter 0,5 m/sec und insbesondere bei 0,25 m/sec liegt.

3. Verfahren nach Anspruch 1 und 2, **dadurch gekennzeichnet, daß** bei einem Rohrinnendurchmesser von 205 mm ein Luftvolumenstrom von 10 bis 50, insbesondere 30 m³/h eingestellt wird.

4. Verfahren nach einem oder mehreren der Ansprüche 1 bis 3, **dadurch gekennzeichnet, daß** eine Außenfläche von 3 bis 5, insbesondere 4 cm² bei einer Wandstärke des Stahlrohres im Bereich von 6,9 mm auf Rotglut erhitzt wird.

5. Verfahren nach Anspruch 4, **dadurch gekennzeichnet, daß** bei dickeren Wandstärken die zu erhitzende Außenfläche zunimmt.

6. Verfahren nach einem oder mehreren der vorstehenden Ansprüche 1 bis 5, **dadurch gekennzeichnet, daß** man die vorgegebene Außenfläche des Stahlrohres innerhalb von 20 bis 40, insbesondere 30 sec bis zur Rotglut erhitzt; wobei eine Oberflächentemperatur außen von ca. > 800°C erreicht wird.

7. Verfahren nach einem oder mehreren der vorstehenden Ansprüche 1 bis 6, **dadurch gekennzeichnet, daß** die kontaminierten Stahlrohre auf einen Zielwert von < 5 mg Hg/kg Stahl gereinigt werden.

## Claims

1. Method for determining the mercury content of contaminated steel pipe sections and/or fittings, which are in the form of natural gas pipes, which are both in the ground and can be dismantled, which is performed in situ in the field with the following steps:
a) initial scavenging of the contaminated steel pipe section or fittings to be investigated using fresh air, in order to obtain a complete air replacement therein,
b) setting a predetermined, constant laminar air flow,
c) heating a clearly defined, punctiform outer surface of the steel pipe section until red hot and its corresponding inner surface to > 100°C with a constant energy introduction into the pipe section, to obtain an evaporation of the mercury which has collected on the inner surface and in the structure of the steel pipe section,
d) continuous sucking off of the air flow with the mercury absorbed by it and supplied to a measuring device,
e) measuring the liberated, elementary mercury content in the sucked off air flow in per se known manner by atomic absorption spectroscopy in the gas phase,
f) recording the liberated mercury content over a short time period and establishing the maximum value,
g) calculating the mg/Hg content/kg of steel pipe section on the basis of the linearity existing with the mg/Hg content in the established air flow quantity.

2. Method according to claim 1, **characterized in that** the constant air flow is provided by an exhaust fan and is in particular below 0.5 m/sec and in particular at 0.25 m/sec.

3. Method according to claim 1 and 2, **characterized in that** with a pipe internal diameter of 205 mm, an air volume flow of 10 to 50, particularly 30 m³/h is set.

4. Method according to one or more of the claims 1 to 3, **characterized in that** an outer surface of 3 to 5, particularly 4 cm² in the case of a steel pipe wall thickness of approximately 0.69 mm is heated red hot.

5. Method according to claim 4, **characterized in that** the outer surface to be heated increases with greater wall thicknesses.

6. Method according to one or more of the preceding claims 1 to 5, **characterized in that** the predetermined outer surface of the steel pipe is heated to red hot within 20 to 40 and in particular 30 sec, an external surface temperature of approx. > 800°C being reached.

7. Method according to one or more of the preceding claims 1 to 6, **characterized in that** the contaminated steel pipes are cleaned to a target value of < 5 mg Hg/kg steel.

## Revendications

1. Procédé pour déterminer la teneur en mercure dans des sections de tuyau et/ou armatures en acier contaminées, qui sont des conduites de gaz naturel, pouvant aussi bien se trouver dans le sol qu'être démontées, ledit procédé étant exécuté sur le terrain et sur place et comportant les étapes suivantes consistant :
a) à arroser d'abord d'air frais la section de tuyau et/ou des armatures en acier à analyser, pour obtenir l'entier renouvellement d'air désiré dans celles-ci,
b) à régler un flux d'air laminaire prédéterminé constant,
c) à chauffer une surface externe ponctuelle et définie de la section du tuyau en acier jusqu'à rougissement et la face interne correspondante à une température > 100°C en fournissant une énergie constante dans la section du tuyau pour obtenir une évaporation du mercure accumulé sur la face interne et dans la structure de la section de tuyau en acier,
d) à aspirer en continu le flux d'air avec le mercure qu'il a absorbé et à le conduire dans un dispositif de mesure,
e) à mesurer la teneur en mercure élémentaire dégagé dans le flux d'air aspiré d'une manière connue en elle-même au moyen de la spectroscopie d'absorption atomique en phase gazeuse,
f) à enregistrer de la teneur en mercure dégagé sur une courte période et à constater la valeur maximale,
g) à calculer le contenu mg/Hg par kg de section de tuyau en acier sur la base de la linéarité existante par rapport au contenu mg/Hg dans la quantité de flux d'air déterminée.

2. Procédé selon la revendication 1, **caractérisé en ce que** le flux d'air constant est envoyé au moyen d'un ventilateur de tirage par aspiration, ledit flux se situant en dessous de 0,5 m/sec et notamment aux environs de 0,25 m/sec.

3. Procédé selon la revendication 1 et 2, **caractérisé en ce que** l'on règle pour un diamètre interne de tuyau de 205 mm le débit d'air entre 10 et 50, en particulier à 30 m³/h.

4. Procédé selon l'une ou plusieurs des revendications 1 à 3, **caractérisé en ce qu'**une surface externe de 3 à 5, notamment de 4 cm² pour une épaisseur de tuyau en acier de 6,9 mm est chauffée jusqu'à rougissement.

5. Procédé selon la revendication 4, **caractérisé en ce que** la surface externe à chauffer augmente si la paroi est plus épaisse.

6. Procédé selon l'une ou plusieurs des revendications précédentes 1 à 5, **caractérisé en ce que** l'on chauffe la surface externe prédéterminée du tuyau en acier pendant entre 20 et 40, particulièrement 30 secs jusqu'à rougissement, une température superficielle externe environ > 800°C étant atteinte.

7. Procédé selon l'une ou plusieurs des revendications précédentes 1 à 6, **caractérisé en ce que** les tuyaux en acier contaminés sont purifiés à une valeur cible de 5 mg Hg/kg d'acier.
